# EUROPÄISCHE PATENTSCHRIFT

(11) **EP 1 078 292 B1**
(45) Veröffentlichungstag und Bekanntmachung des Hinweises auf die Patenterteilung: **13.06.2007**
(21) Anmeldenummer: 00912512.1
(22) Anmeldetag: 26.02.2000
(51) Int. Cl.: G02B 23/24, A61B 1/00

(54) **ENDOSKOPOPTIK MIT LINSENVERSTELLSICHERUNGSEINRICHTUNG**
ENDOSCOPIC OPTICAL SYSTEM WITH SAFETY DEVICE FOR PREVENTING THE DISPLACEMENT OF LENSES
INSTRUMENT OPTIQUE ENDOSCOPIQUE DOTE D'UN DISPOSITIF DESTINE A EMPECHER LE DEPLACEMENT DES LENTILLES

(30) Priorität: 20.03.1999 DE 19912656
(43) Veröffentlichungstag der Anmeldung: 28.02.2001
(73) Patentinhaber: OLYMPUS WINTER & IBE GMBH, 22045 Hamburg (DE)
(72) Erfinder: FRISCHE, Holger, D-21244 Buchholz (DE); WEBER, Michael, D-20148 Hamburg (DE); WULFSBERG, Jens, Peter, D-23843 Neritz (DE)
(74) Vertreter: Schaefer, Konrad
(86) Internationale Anmeldenummer: PCT/EP2000/001610
(87) Internationale Veröffentlichungsnummer: WO 2000/057232

(56) Entgegenhaltungen:
- DE-A- 2 150 595
- DE-A- 3 839 364
- DE-A- 3 912 720
- DE-A- 4 442 185
- DE-A- 19 732 991
- GB-A- 2 099 174
- US-A- 3 946 727
- US-A- 4 148 550
- US-A- 5 760 976

## Beschreibung

Die Erfindung betrifft eine Endoskopoptik der im Oberbegriff des Anspruches 1 genannten Art.

Derartige Endoskopoptiken werden insbesondere in der medizinischen Endoskopie verwendet. Sie weisen eine Anordnung mehrerer Linsen auf. üblicherweise eine Anordnung von Stablinsen als Relaisoptik sowie an deren distalem Ende eine Anordnung von Objektivlinsen, die üblicherweise mit den Stablinsen als integrierte Baueinheit vorgesehen sind. Ferner sitzt am proximalen Ende der Stablinsenanordnung ein Okular, das üblicherweise gesondert von der Stablinsenanordnung montiert ist. Die Stablinsen und das Objektiv sind üblicherweise in einem Systemrohr gelagert, das die Ausrichtung der Linsen gewährleistet und zu deren gasdichter Abdichtung mit Fenstern verwendbar ist, wie sie bei der Heißdampfsterilisierung für medizinische Anwendungen erforderlich ist.

Solche Endoskopoptiken sollen möglichst biegsam sein, um in Körperkanälen auch leicht gebogen verlegt werden zu können. Dabei müssen die empfindlichen Stablinsen gegen Bruch gesichert sein. Bei Anordnung der Linsen in einem Systemrohr wird üblicherweise ein radiales Spiel vorgesehen, damit die Linsen beim Biegen des Systemrohres nicht brechen.

Die Linsen müssen in ihrer relativen Lage zueinander gesichert sein, um eine gute optische Qualität der Endoskopoptik zu sichern. Das bedeutet, daß die Linsen in definierten Achsabständen zueinander fixiert und auch radial fixiert sein müssen. Dazu sind Sicherungseinrichtungen bekannt. Üblicherweise werden die Linsen radial gegenüber dem Systemrohr abgestützt und axial durch zwischen den Linsen angeordnete Distanzrohre beabstandet.

Bei Stoßbetastungen der Endoskopoptik, beispielsweise wenn diese vom Tisch auf den Fußboden fällt, können sich die Linsen durch einwirkende Beschleunigungskräfte verschieben. Sie können dabei verkippen, was zu einem "Verspringen" der Blickrichtung der Optik führt. Sie können sich dabei auch verdrehen, was ebenfalls zu einem Verspringen der Blickrichtung führt, da in Großserie hergestellte Stablinsen zumeist eine Abweichung zwischen optischer Achse und geometrischer Achse aufweisen.

Um dies zu verhindern, ist eine Sicherungseinrichtung erforderlich. Eine solche Sicherungseinrichtung ergibt sich aus der nachveröffentlichten deutschen Patentanmeldung 19742454.6-51. Bei dieser Konstruktion werden die Stablinsen mit speziellen Distanzrohren und unter gewollter Schrägstellung in stabiler Lage angeordnet, in die sie auch nach Stoßerschütterungen stets wieder zurückkehren. Linsenverdrehungen werden mit dieser Sicherungseinrichtung jedoch nur ungenügend verhindert.

Eine Konstruktion gemäß DE 39 12 720 C2 ersetzt das Systemrohr durch einen Schrumpfschlauch, der vor Schrumpfung Übermaß aufweist, zum leichten Einfüllen der Linsen, und nach Schrumpfung diese radial fest schließend sichert. Dadurch ergibt sich eine dauerhaft gute Ausrichtung der Linsen zueinander, wobei diese auch drehgesichert sind. Nachteilig hierbei ist jedoch der aufwendige Schrumpfvorgang, sowie die Notwendigkeit, bestimmte schrumpffähige Kunststoffe verwenden zu müssen, die andere störende Materialeigenschaften aufweisen. Ferner läßt sich eine solche Optik später nicht mehr reparieren, sondern kann nur als Ganzes getauscht werden. Außerdem ist das verwendete Schrumpfmaterial thermisch empfindlich und für die Heißdampfsterilisation in der Regel nicht geeignet. Vor allem ist bei diesem Material aus Gründen von Materialabweichungen, Fertigungsabweichungen oder Effekten späterer thermischer Einwirkung die dauerhaft feste Sicherung der Linsen nicht gewährleistet. Läßt die Haltekraft des Materiales etwas nach, sind die Linsen lose und können sich verschieben.

Die US 4,148,550 A zeigt eine gattungsgemäße Endoskopoptik mit einem die Linsen elastisch rückfedern haltenden Rohr, das in Ruhestellung oval ist und entgegen seiner Rückfederkraft in eine runde Stellung bringbar ist, bei der die Linsen justierbar sind. Dabei kann das Rohr zusätzlich thermisch aufgeweitet werden.

Die Aufgabe der vorliegen Erfindung besteht darin, eine gattungsgemäße Endoskopoptik mit einer dauerhaft die Linsen sichernden, leicht montierbaren und bei Bedarf reparierbaren Sicherungseinrichtung vorzusehen.

Diese Aufgabe wird mit den Merkmalen des Anspruches 1 gelöst.

Die Erfindung geht von der Erkenntnis aus, daß es zur Stoßsicherheit der Optik ausreicht, jeweils an den Stoßstellen die Linsenenden gegeneinander fluchtend und drehgesichert zu halten. Erfindungsgemäß geschieht dies mit einem umfangsaufweitbaren, an wenigstens einer Stoßstelle vorgesehenem Rohr, das aus einem engeren Durchmesser dauerrückfedernd aufgeweitet die Linsenenden faßt und das zum Zwecke der bruchsicheren Biegsamkeit der Optik mit Spiel im Systemrohr gelagert sein kann. Ein solches Rohr kann materialschonend bei Stößen leicht federnd nachgeben und auch thermische Ausdehnungen der Linsen zulassen, hält die Linsen aber stets zueinander axial und drehsichernd ausgerichtet. Bei aufgeweitetem Rohr können die Linsen leicht montiert oder zu Reparaturzwecken ausgewechselt werden. Das die Linsen aneinander sichernde Rohr hält diese im Sinne einer Montageeinheit zusammen, so daß Linsenpaare oder die gesamte Linsenanordnung der Endoskopoptik, durch die Sicherungseinrichtung zusammengehalten, als Montageeinheit gehandhabt werden kann, beispielsweise in ein Systemrohr oder einen sonstigen Montageort eingeführt werden kann. Da die radial federnde Sicherungseinrichtung auch axial festhält, können den Achsabstand sichernde Distanzrohre zwischen den Linsen eingespart werden.

Ein sicherndes Rohr kann nur an der Stoßstelle zweier Linsen diese aneinander sichern, um die wesentliche optische Ausrichtung der Linsen zu gewährleisten. Das Rohr kann sich vorteilhaft gemäß Anspruch 2 aber auch vollständig über die gesicherten Linsen erstrecken, um diese noch besser gegen gegenseitige Verkippung zu sichern.

Das Rohr kann vorteilhaft nach Anspruch 3 als Schlauch aus umfangsdehnelastischem Material. z.B. einem gummiähnlichen Kunststoffmaterial ausgebildet sein. Zum Einfüllen des Linsenstapels muß dieser Schlauch umfangsaufgeweitet werden. Dies kann z.B. durch Unterdrucksetzung von Innen mit Preßluft oder vorteilhaft durch Anlegen von Vakuum auf der Außenseite des Schlauches erfolgen.

Alternativ kann das Rohr auch gemäß Anspruch 4 als Ziehstrumpf ausgebildet sein, wie er beispielsweise aus DE 19630666 A1 bekannt ist. Ein solcher Ziehstrumpf kann in Engstellung hergestellt sein. Er muß dann zur Linsenmontage durch axialen Druck aufgeweitet werden. Nach Loslassen zieht er sich wieder in Engstellung sichernd um die Linsen. In anderer Ausbildung kann er auch weitgestellt gefertigt sein. Er wird dann nach Einfüllen des Linsenstapels unter axialen Zug gesetzt, so daß er sich engstellend um die Linsen schließt. Er muß dann in endgültiger Montagestellung im Systemrohr unter Zug gehalten bleiben.

Alternativ ist gemäß Anspruch 5 das Rohr als längsgeschlitztes Rohr aus biegeelastischem Material ausgebildet. Zum Einfüllen des Linsenstapels wird das Rohr an seinem Schlitz aufgeweitet. Nach Loslassen schließt es sich radial federnd um die Linsen.

Vorteilhaft ist ein solches Rohr gemäß Anspruch 6 als Flachdrahtwendel ausgeführt Eine solche Flachdrahtwendel hat zunächst enorme Kostenvorteile. Sie hat außerdem wesentliche Vorteile für die Montage. Eine in Engstellung hergestellte Flachdrahtwendel kann durch Anfassen an den Enden und gegenläufiges Verdrehen aufgeweitet werden zum Einfüllen der Linsen. Nach Loslassen umschließt sie diese radial nach innen federnd mit hervorragender Sicherungswirkung. In der vorteilhaften Ausbildung aus Metall ist sie hervorragend für langdauernde Haltbarkeit unter Autoklavierbedingungen geeignet. Ein wesentlicher Vorteil der Flachdrahtwendel liegt ferner darin, daß sie nach Drehaufweiten und Loslassen nicht nur radial, sondern auch in Längsrichtung einfedert. Sie sorgt also nicht nur für Verkipp-und Drehsicherung der Linsen, sondern auch für deren axial federnde Anlage, ohne daß dafür eine gesonderte Einrichtung erforderlich wäre Wenn auf diese Weise der Linsenstapel axial vorgespannt wird, sind zur Sicherung der axialen Abstände zwischen den Linsen gesonderte Einrichtungen. z.B. in Form der üblichen Distanzrohre erforderlich.

Die Rohre gemäß Anspruch 5 oder Anspruch 6 können aus biegefederndem Material wie z.B. Kunststoff, bestehen. Vorteilhaft sind sie jedoch gemäß Anspruch 7 aus Metall ausgeführt (z.B. Federstahl). In Metallausführung ergibt sich eine langfristig haltbare und thermisch (z.B. beim Autoklavieren) sehr funktionssichere Konstruktion.

Vorteilhaft weist ein geschlitztes Rohr nach Anspruch 8 einen achsparallelen Schlitz auf. Dies ergibt eine einfache Konstruktion, bei der der Schlitz mit einfachen Mitteln zum Einsetzen der Linsen geöffnet werden kann.

Ein solcher Schlitz kann sehr schmal sein, so daß das Rohr in Sicherungsstellung die Linsen fast vollständig umschließt. Vorteilhaft sind jedoch die Merkmale des Anspruches 9 vorgesehen. Ein sehr breiter Schlitz, bei dem also das Rohr die Linsen nur um etwas mehr als 180° umfaßt gewährleistet noch immer eine sichere Fassung der Linsen durch das sichernde Rohr, erlaubt aber bei geringfügiger Aufweitung des Schlitzes das seitliche Einlegen der Linsen, die also nicht, wie in der üblichen Montagetechnik, von den Enden des Rohres her eingeschoben werden müssen. Durch den weit offenen Schlitz lassen sich ferner die Linsen durch einfachen seitlichen Eingriff im Rohr genau justieren, und zwar sowohl in Achsrichtung als auch in Drehrichtung, um beispielsweise bei Linsen mit Abweichungen zwischen der optischen und er geometrischen Achse diese untereinander durch Drehung optimal anzupassen.

Das erfindungsgemäße Rohr kann, wie bereits erwähnt, nur an Stoßstellen je zwei Linsen aneinander koppeln oder einzelne Linsengruppen koppeln. Vorzugsweise sind jedoch die Merkmale des Anspruches 10 vorgesehen. Damit können alle Linsen der Endoskopoptik in einem Rohr montiert, ausgerichtet und gesichert und bei entsprechender Ausbildung des Rohres. z.B. als durchgehendes längsgeschlitztes stabiles Rohr, als Montageeinheit weiterverarbeitet werden.

Vorteilhaft sind die Merkmale des Anspruches 11 vorgesehen. Das erfindungsgemäß sichernde Rohr kann z.B. bei einem mehrlinsigen Objektiv dessen Linsen gegeneinander sichern oder auch das Objektiv gegenüber der benachbarten Stablinse sichern. Bei Anordnung sämtlicher Linsen der Endoskopoptik in einem Rohr sind hierbei vorteilhaft auch die Objektivlinsen in derselben Anordnung ausgerichtet und gesichert.

Bei einer Endoskopoptik mit Systemrohr sind vorteilhaft die Merkmale des Anspruches 12 vorgesehen. Das erfindungsgemäße umfangsaufweitbare Rohr sichert die Linsen gegen axiale und/oder radiale Verschiebung insbesondere bei Stoßbelastung und liegt im Montagezustand den Linsen federnd unmittelbar an. Um beim Biegen des Systemrohres Biegebelastung der Linsen zu vermeiden, ist das radiale Spiel vorgesehen, mit dem eine gewisse Biegung des Systemrohres ohne Bruchbelastung der Linsen ermöglicht wird.

In den Zeichnungen ist die Erfindung beispielsweise und schematisch dargestellt. Es zeigen:
- Fig. 1: einen Längsschnitt durch eine erfindungsgemäße Endoskopoptik mit Darstellung eines eine Stoßstelle sichernden Rohrstückes.
- Fig. 2a: in perspektivischer Darstellung eine Sicherungseinrichtung gemäß Fig. 1 in Ausführung als geschlitztes Metallrohr.
- Fig. 2b: in perspektivischer Darstellung mehrere untereinander mit Stegen längsverbundene Rohre gemäß Fig. 2a.
- Fig. 3: ein perspektivische Seitendarstellung eines elastischen Schlauches.
- Fig. 4a, 4b: ein perspektivische Seitendarstellung einer Sicherungseinrichtung in Form eines Ziehstrumpfes in Weitstellung (Fig. 4a) und Engstellung (Fig. 4b).
- Fig. 5: ein perspektivische Seitendarstellung einer Sicherungseinrichtung in Form eines langgestreckten, geschlitzten Metallrohres.
- Fig. 6: ein Seitendarstellung einer Sicherungseinrichtung in Form einer Flachdrahtwendel.
- Fig. 7: einen Längsschnitt durch eine Endoskopoptik mit Sicherungseinrichtung gemäß Fig. 6.
- Fig. 8: einen Querschnitt durch eine Endoskopoptik mit Systemrohr und Sicherungseinrichtung in Form eines breit geschlitzten Rohres,
- Fig. 9: den Schnitt der Fig. 8, jedoch ohne Systemrohr und mit aufgeweitet gehaltenem Schlitz und
- Fig. 10: einen Längsschnitt nach Linie 10 - 10 in Fig. 8.

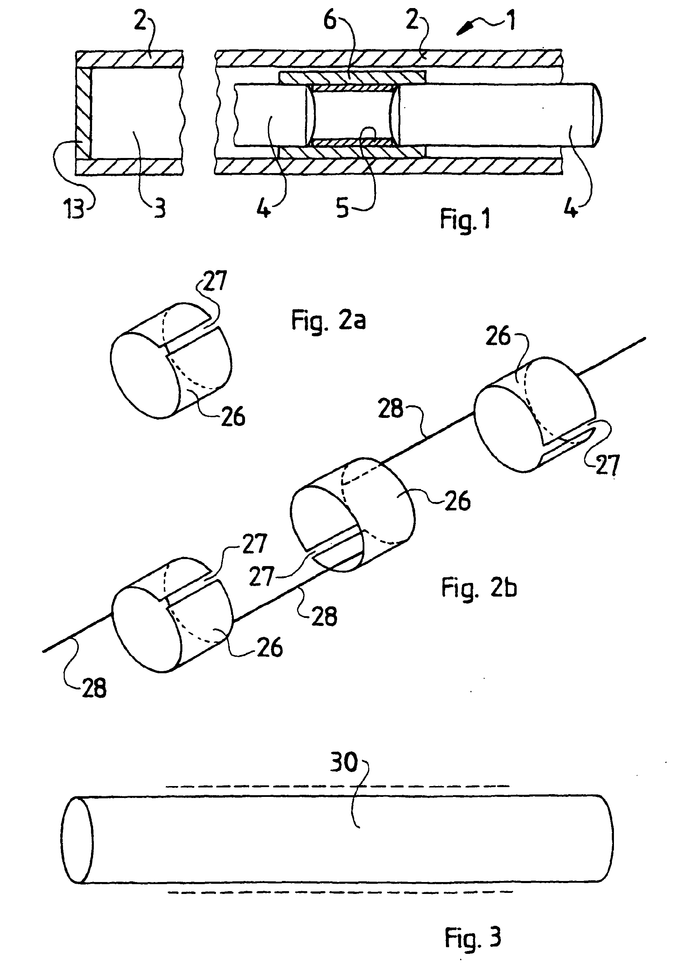

Fig. 1 zeigt im Längsschnitt eine Endoskopoptik 1 mit einem aus Metall bestehenden Systemrohr 2, das an den Enden, von denen nur eines dargestellt ist, beispielsweise mit dem dargestellten Fenster 13 gasdicht verschlossen ist. In dem somit gasdicht umschlossenen Innenraum 3 des Systemrohres 2 ist zur Bildübertragung von einem nicht dargestellten, an einem Ende der Optik angeordneten Objektiv, zu einem nicht dargestellten, am anderen Ende der Optik vorgesehenen Okular bzw. einer dort vorgesehenen Kamera, ein Stapel von Stablinsen 4 angeordnet, von denen in Fig. 1 zwei benachbarte Linsen dargestellt sind. In üblicher Ausführung sind die Stablinsen jeweils mit einem Distanzrohr 5 axial aufeinander abgestützt. Eine nicht dargestellte, an einem Ende des Systemrohres 2 vorgesehene, in Achsrichtung wirkende Feder setzt in üblicher Bauweise den Linsenstapel unter axiale Vorspannung, damit alle Linsen und Distanzrohre axial aneinanderliegend gehalten sind.

Die Stablinsen 4 sind, wie dargestellt, mit radialem Spiel zum Systemrohr 2 angeordnet. Dieses kann daher auch über engere Radien gebogen werden, ohne daß dabei die empfindlichen Stablinsen 4 zerbrechen.

Wird die dargestellte Endoskopoptik 1 Stößen ausgesetzt, so bewegen sich ihre Einzelteile aufgrund ihrer Massenträgheit gegeneinander. An der dargestellten Stoßstelle kann es dabei zu Radialverschiebungen der Linsenenden zueinander kommen, oder auch zu Rotationsverdrehungen der Linsen 4, bei denen nicht unbedingt die optische Achse mit der geometrischen Achse übereinstimmt. Beide Verschiebungen führen zu einem Verspringen der optischen Achse. Bereits ein solches Verspringen an einer Stoßstelle und erst recht an mehreren Stoßstellen, führt zu einem deutlich störenden Verspringen der Blickrichtung der Optik.

Um dies zu verhindern, ist eine Sicherungseinrichtung vorgesehen, die in dem in Fig. 1 dargestellten Ausführungsbeispiel aus einem Rohrstück 6 besteht, welches an der dargestellten Stoßstelle das Distanzrohr 5 und die Endbereiche der beiden angrenzenden Stablinsen 4 überfaßt. Das Rohrstück 6 ist dauerrückfedernd umfangsaufweitbar ausgebildet. Unter Berücksichtigung nur der angestrebten Sicherungsfunktion könnte es als Stück Gummischlauch ausgebildet sein, das aufgeweitet über die Stoßstelle geschoben und dann losgelassen wird. Für die angestrebten Autoklavierzwecke wäre geeignet federndes Kunststoffmaterial verwendbar. Da das Rohrstück 6 die beiden gesicherten Linsen 4 federnd ausreichend festhält, kann auf das Distanzrohr 5 auch verzichtet werden und die axiale Sicherung der beiden Linsen gegeneinander dem federnden Rohrstück 6 überlassen werden.

Fig. 2a zeigt ein anstelle des Rohrstückes 6 in Fig. 1 verwendbares Rohrstück 26 aus biegeelastischem Material, vorzugsweise aus Metall, mit einem zwischen den Rohrenden durchgehend verlaufenden Längsschlitz 27. Das Rohrstück 26 ist mit kleinerem Innendurchmesser als der Außendurchmesser der Stablinsen 4 vorgefertigt, wird an seinem Schlitz 27 aufgeweitet, dann in die Position des Rohrstükkes 6 gemäß Fig. 1 gebracht und losgelassen. Es umschließt dann federnd sichernd die Endbereiche der an der dargestellten Stoßstelle aneinandergrenzenden Stablinsen 4.

In Fig. 2b ist dargestellt, daß mehrere geschlitzte Rohrstücke 26 gemäß Fig. 2a mit Stegen 28 verbunden sein können. Sie werden von den Stegen im Abstand der Stoßstellen, an denen die hintereinanderliegenden Stablinsen jeweils aneinander stoßen, gehalten. Es ergibt sich dadurch eine Montageeinheit z.B. aller benötigten Rohrstücke 26 für ein Stablinsensystem. Außerdem wird die axiale Lage der Rohrstücke 26 an den Stoßstellen sichergestellt. Wie dargestellt, können die Schlitze 27 der Rohrstücke 26 und die Stege 28 zueinander winkelversetzt sein um der Gesamtanordnung eine in allen Richtungen gleich gute Biegsamkeit zu verleihen. Die Montageeinheit der Rohrstücke 26 ergibt mit eingesetzten Linsen eine Montageeinheit der Linsenanordnung, die auch ohne umhüllendes Rohr, beispielsweise zu Montagezwecken, handhabbar ist.

Fig. 3 zeigt eine Sicherungseinrichtung in Form eines langgestreckten Schlauches 30, der aus dauerelastisch umfangsdehnbarem Material besteht, wie es bereits zum Rohrstück 6 gemäß Fig. 1 erwähnt wurde. Der Schlauch 30 erstreckt sich jedoch über die gesamte Länge der Endoskopoptik 1 hinweg, also über alle Stoßstellen hinweg, um diese zu sichern. Der Schlauch 30 hat in dem mit ausgezogenen Linien dargestellten Ruhezustand einen Innendurchmesser kleiner als der Außendurchmesser der Stablinsen 4, die in Fig. 3 zur zeichnerischen Vereinfachung nicht dargestellt sind. Er kann elastisch aufgeweitet werden, bis zu dem mit gestrichelten Linien angedeuteten größeren Durchmesser. Dann kann das Stablinsensystem eingefüllt werden. Nach Loslassen des Schlauches 30 zieht sich dieser radial zusammen, um die Stablinsen zu sichern. Das Aufweiten kann beispielsweise durch Aufblasen mit Preßluft von Innen oder vorzugsweise durch Anlegen von Vakuum von Außen mit geeigneten Montagehilfseinrichtungen erfolgen.

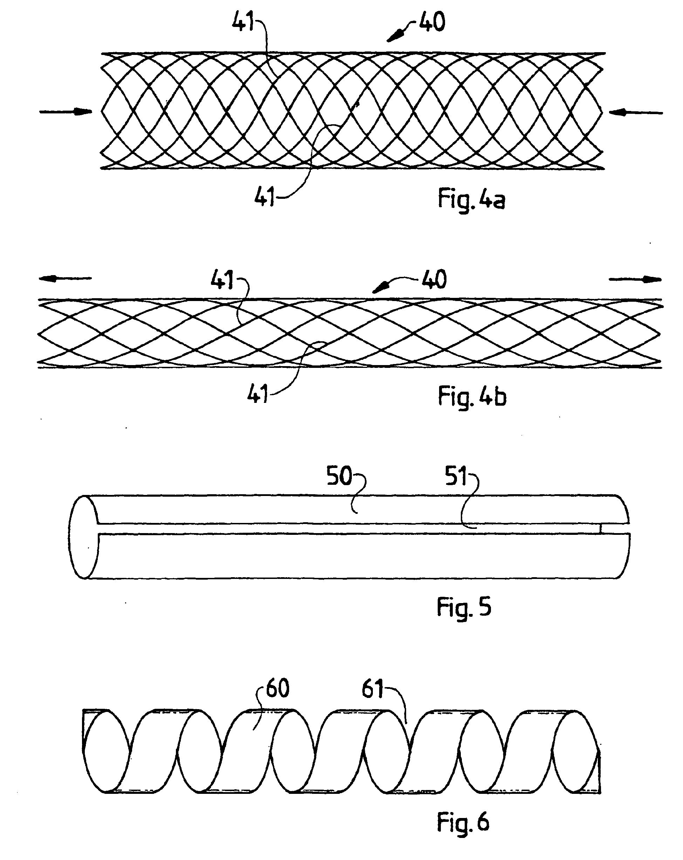

Die Fig. 4a und 4b zeigen als Sicherungseinrichtung, die denselben Zweck erfüllt, wie die in Fig. 3 dargestellte Einrichtung, einen Ziehstrumpf 40, der aus einer Vielzahl von gekreuzt angeordneten Fasern 41 besteht. An den Kreuzungsstellen der Fasern können diese verflochten oder auch vernetzt sein. Ein solcher Ziehstrumpf hat die Eigenschaft, bei axialem Zusammendrücken in Pfeilrichtung gemäß Fig. 4a aufzuweiten und bei axialem Zug in Pfeilrichtung gemäß Fig. 4b seinen Umfang zu verringern.

Der Ziehstrumpf 40 kann in Engstellung gemäß Fig. 4b vorgefertigt sein. Erläßt sich dann in die Weitstellung gemäß Fig. 4a axial zusammendrücken. In dieser Stellung wird der nicht dargestellte Linsenstapel eingeführt. Dann wird losgelassen, und der Ziehstrumpf 40 zieht sich elastisch in die Stellung gemäß Fig. 4b im Umfang zusammen unter Sicherung des Linsenstapels. In anderer Ausführung kann der Ziehstrumpf 40 auch in Weitstellung gemäß Fig. 4a vorgefertigt sein. Nach Einfüllen des Linsenstapels wird er gemäß Pfeilen in Fig. 4b unter axialen Zug gesetzt, um sich elastisch um die Linsen zu schließen. Er muß dann in dem Systemrohr 2, wie es in Fig. 1 dargestellt ist, unter Aufrechterhaltung der Zugspannung montiert werden.

Fig. 5 zeigt eine weitere Ausführungsform einer Sicherungseinrichtung, die aus einem langgestreckten Rohr 50 mit Längsschlitz 51 besteht. Dieses Rohr ist grundsätzlich so aufgebaut wie das Rohrstück 26 der Fig. 2a, jedoch langgestreckt über z.B. die gesamte Länge der Optik, sichert also gleichzeitig alle Stoßstellen zwischen Stablinsen. Das Rohr 50 besteht vorzugsweise wiederum aus Federmetall und kann nach Aufweiten des Längsschlitzes 51 mit dem Linsenstapel befüllt werden, den es anschließend nach Umfangsrückfederung sichernd umfaßt.

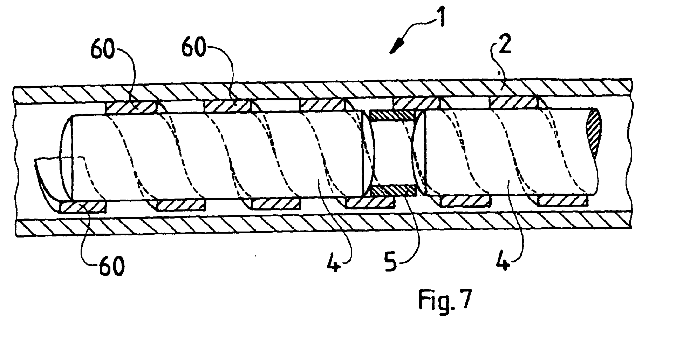

Eine besonders vorteilhafte Ausführungsform ist in den Fig. 6 und 7 dargestellt.

Die Sicherungseinrichtung besteht hier aus einer Flachdrahtwendel 60. Sie umschließt den nicht dargestellten Linsenstapel ebenso, wie beispielsweise das Rohr 50 der Fig. 5 oder der Schlauch 30 der Fig. 3. Die Flachdrahtwendel 60 ist wiederum über die gesamte Länge des Linsenstapels ausgebildet, kann zu speziellen Zwecken aber auch nur einen Teilbereich von dessen Länge überfassen.

Die Flachdrahtwendel 60 der Fig. 6 besteht wie das Rohr 50 der Fig. 5 vorzugsweise aus elastisch federndem Metall. z.B. Federstahl geeigneter Korrosionsbeständigkeit und weist ebenso wie das Rohr 50 der Fig. 5 einen zwischen den Enden durchlaufenden Schlitz 61 auf, der jedoch schraubenförmig verläuft, so daß sich die Flachdrahtwendel 60 ergibt. Die Flachdrahtwendel 60 der Fig. 6 ist in Fig. 7 in Montagestellung im Systemrohr 2 der in Fig. 1 dargestellten Endoskopoptik 1 dargestellt. Es ist in Fig. 7 ein Abschnitt des Systemrohres 2 dargestellt, an dem zwei Stablinsen 4 über ein Systemrohr 5 aneinander stoßen.

Die Flachdrahtwendel 60 ist mit innerem Untermaß gegenüber dem Außenmaß der Stablinsen 4 vorgefertigt. Zum Einfüllen des Linsen/Distanzrohr-Stapels muß sie zunächst federnd aufgeweitet werden. Dazu kann sie einfach an beiden Enden angefaßt werden, beispielsweise mit einer geeigneten Vorrichtung, und es werden die beiden Enden gegenläufig zur Aufweitung der Flachdrahtwendel 60 gedreht. Dabei ergibt sich nicht nur eine Umfangsaufweitung, sondern auch eine axiale Längung der Flachdrahtwendel. Im aufgeweiteten Zustand wird der Linsenstapel eingefüllt und die Flachdrahtwendel wird losgelassen. Sie federt dann in Umfangsrichtung ein sowie auch in Längsrichtung Wie Fig. 7 zeigt, wird mit der Flachdrahtwendel 60 an jeder Stoßstelle eine umgreifende Sicherung der aneinanderstoßenden Enden zweier Stablinsen 4 erreicht. Durch die Längsrückfederung der Flachdrahtwendel 60 ergibt sich auch die gewünschte axilalfedernde Belastung des Linsenstapels, so daß die üblichen, für diesen Zweck vorgesehenen Endfedern entfallen können. Bei dieser Anordnung mit axial rückfedernder Flachdrahtwendel 60 sind zur Sicherung des axialen Abstandes der Linsen 4 zwischen diesen die dargestellten Distanzrohre 5 erforderlich.

Wie Fig. 7 zeigt, ist die Flachdrahtwendel 60 mit leichtem Untermaß ihres Außendurchmessers gegenüber dem Innendurchmessers des Systemrohres 2 vorgesehen, so daß bei Biegung des Systemrohres 2 die empfindlichen Stablinsen 4 vor Zerbrechen geschützt sind.

Die in den Figuren 5 und 6 dargestellten Einrichtungen können sich, wie dargestellt, über die Länge des gesamten zu sichernden Linsenstapels erstrecken. Sie können jedoch auch kürzer ausgebildet sein. So kann das Rohr 50 der Fig. 5 z.B. wie in Fig. 2a dargestellt, sehr kurz nur zur Sicherung einer Linsenstoßstelle ausgebildert sein. Das gilt auch für die Flachdrahtwendel 60 der Fig. 6. die ebenfalls sehr kurz zur Sicherung nur einer Stoßstelle oder etwas länger zur Sicherung mehrerer Stoßstellen ausgebildet sein kann.

Das Rohr 50 der Fig. 5 oder die Flachdrahtwendel 60 der Fig. 6 können jedoch auch mehrstückig zusammengesetzt ausgebildet sein. Dabei kann bei dem Rohr 50 der Fig. 5 in den unterschiedlichen aneinandergesetzten Stücken der Schlitz 51 umfangsversetzt angeordnet sein, ähnlich wie in Fig. 2b dargestellt. Bei der Flachdrahtwendel 60 der Fig. 6 kann bei mehrstückiger Ausbildung z.B. in den einzelnen Stücken die Wendelrichtung alternierend ausgebildet sein. Dies kann ein Vorteil sein, um das Verdrehen der Wendet bei thermischer Ausdehnung des Materials zu verringern.

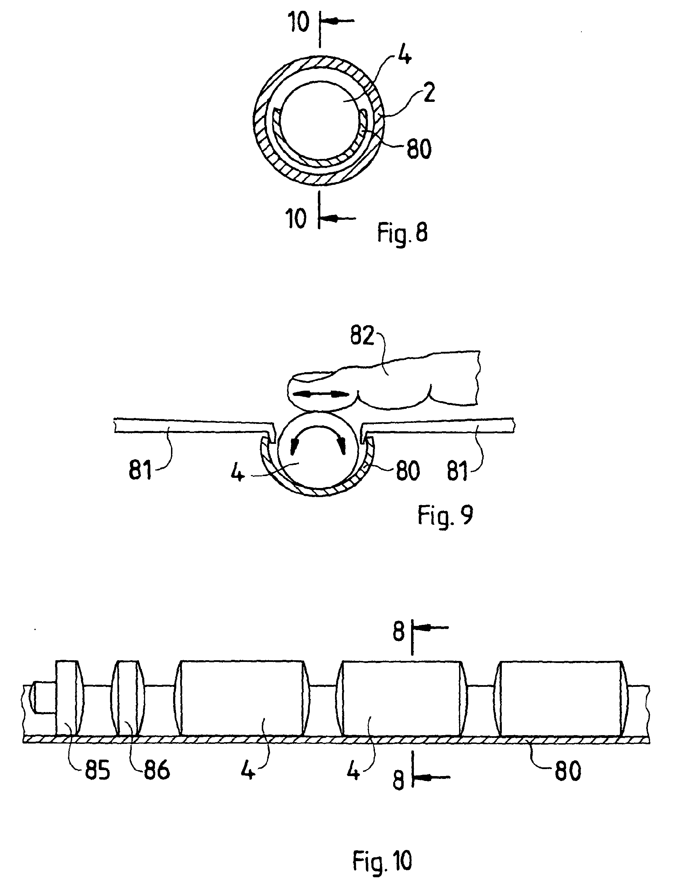

Die Figuren 8 bis 10 zeigen eine weitere vorteilhafte Ausbildung einer Endoskopoptik unter Verwendung eines gerade längsgeschlitzten Rohres 80. das dem Rohr 50 der Figur 5 entspricht bis auf die Tatsache, daß der Schlitz gegenüber dem Schlitz 51 gemäß Figur 5 wesentlich erweitert ist. Er erstreckt sich, wie Figur 8 zeigt, im geschlossenen Zustand des Rohres 80 über etwas weniger als 180° Umfangswinkel. Wie Figur 8 zeigt, hält im geschlossenen Zustand das Rohr 80 die Linsen 4 noch ausreichend umfaßt.

Wie Figur 9 zeigt, kann dieses breit geschlitzte Rohr 80 mit Haltewerkzeugen 81 geöffnet gehalten werden, ohne das Rohr bleibend zu deformieren, so daß die Linsen 4 seitlich eingeschoben werden können, also gemäß Figur 9 von oben wie in eine Wanne eingelegt werden können. Dadurch wird die Montage wesentlich vereinfacht. Bei gemäß Figur 9 eingelegten Linsen 4 sind diese von oben frei zugänglich und können mit dem dargestellten Finger 82 in Achsrichtung bewegt oder, wie mit Pfeilen dargestellt, gedreht werden. Durch gegenseitige Verdrehung der eingelegten Linsen können zum Beispiel unter Überwachung auf einer optischen Bank die Linsen zueinander drehausgerichtet werden. Das ist erforderlich, wenn aus Gründen der Fertigungsqualität bei den Linsen die optische Achse und die geometrische Achse nicht übereinstimmt.

In Figur S ist ersichtlich, daß die Linsen 4 mit dem sichernden Rohr 80 im Systemrohr 2 mit radialem Spiel angeordnet sind, wie dies auch in Figur 7 dargestellt ist, um die Bruchgefahr zu verringern. Figur 8 zeigt jedoch auch, daß bei Verwendung des breit geschlitzten Rohres 80 in dem breiten Schlitz, in dem die Wandstärke des Rohres 80 fehlt, ein zusätzlicher Innenraum zur Verfügung gestellt wird, der in dem beengten Querschnitt einer Endoskopoptik zu beliebigen Nutzungszwecken willkommen ist. Es können hier beispielsweise Beleuchtungsglasfasern, elektrische Leitungen oder dergleichen verlegt werden, für die sonst kein Platz wäre.

Figur 10 zeigt die Anordnung der Figur 8 im Längsschnitt. Es ist hier dargestellt, daß außer den Stablinsen 4 auch zwei Objektivlinsen 85 und 86 im Rohr 80 sichernd gehalten werden, so daß eine hervorrgend stoßgesicherte optische Qualität der gesamten Endoskopoptik gewährleistet werden kann. In anderen, nicht dargestellten Ausführungen kann ein umfangselastisch aufweitbares sicherndes Rohr, wie das Rohr 80, in kürzerer Ausführung auch nur zur Sicherung eines Objektivs mit mehreren Linsen. z.B. mit den Linsen 85 und 86 verwendet werden. Das Rohr kann dann z.B. auch sichernd die erste benachbarte Stablinse fassen, wobei die übrige Stablinsenanordnung zum Beispiel auf andere Weise gesichert ist.

## Patentansprüche

1. Endoskopoptik (1) mit Linsen (4, 85, 86) und mit einer Sicherungseinrichtung (6, 26, 30, 40, 50, 60, 80) zur Lagesicherung der Linsen (4, 85, 86) gegen Verstellung bei Stoßbelastung, die als wenigstens an einer Stoßstelle die aneinandergrenzenden Enden zweier Linsen (4, 85, 86) gemeinsam umfassendes, dauerrückfedernd aufweitbares Rohr (6, 26, 30, 40, 50, 60, 80) ausgebildet ist, **dadurch gekennzeichnet, daß** das Rohr (6, 26, 30, 40, 50, 60, 80) mechanisch gegen die Rückfederkraft umfangsaufweitbar ausgebildet ist.

2. Endoskopoptik nach Anspruch 1, **dadurch gekennzeichnet, daß** das Rohr (30, 40, 50, 60, 80) über die gesicherten Linsen (4, 85, 86) erstreckt ausgebildet ist.

3. Endoskopoptik nach Anspruch 1, **dadurch gekennzeichnet, daß** das Rohr als Schlauch (30) aus umfangsdehnelastischem Material ausgebildet ist.

4. Endoskopoptik nach Anspruch 1, **dadurch gekennzeichnet, daß** das Rohr als Ziehstrumpf (40) aus gekreuzt schräg laufend verwebten oder vernetzten Fasern (41) ausgebildet ist.

5. Endoskopoptik nach Anspruch 1, **dadurch gekennzeichnet, daß** das Rohr (26, 50, 60, 80) aus biegeelastischem Material mit einem die Rohrenden durchgehend verbindenden Schlitz (27, 51, 61) ausgebildet ist.

6. Endoskopoptik nach Anspruch 5, **dadurch gekennzeichnet, daß** das Rohr als Flachdrahtwendel (60) ausgebildet ist.

7. Endoskopoptik nach Anspruch 5, **dadurch gekennzeichnet, daß** das Rohr (26, 50, 60, 80) aus Metall ausgebildet ist.

8. Endoskopoptik nach Anspruch 5, **dadurch gekennzeichnet, daß** der Schlitz (51) des Rohres (26, 50, 80) achsparallel verläuft.

9. Endoskopoptik nach Anspruch 8, **dadurch gekennzeichnet, daß** die Breite des Schlitzes (51) des Rohres (80) kleiner ist als 180° Umfangswinkel.

10. Endoskopoptik nach Anspruch 1, **dadurch gekennzeichnet, daß** das Rohr (30, 40, 50, 60, 80) sämtliche Linsen (4, 85, 86) der Endoskopoptik aufnimmt.

11. Endoskopoptik nach Anspruch 1 mit distal vorgesehenem Objektiv (85, 86) **dadurch gekennzeichnet, daß** das Rohr (80) wenigstens das Objektiv (85, 86) aufnimmt.

12. Endoskopoptik nach Anspruch 1, mit die Linsen (4, 85, 86) aufnehmendem Systemrohr (2), **dadurch gekennzeichnet, daß** das umfangsaufweitbare Rohr (6, 60, 80) mit radialem Spiel im Systemrohr (2) angeordnet ist.

## Claims

1. Endoscope optics (1) comprising lenses (4, 85, 86) and a securing-device (6, 26, 30, 40, 50, 60, 80) for securing the position of the lenses (4, 85, 86) against displacement under shock-impact, the securing-device being designed as a permanently back-springing expandable tube (6, 26, 30, 40, 50, 60, 80) grasping at at least one abutting-site the adjacent ends of two lenses (4, 85, 86), **characterized in that** the tube (6, 26, 30, 40, 50, 60, 80) is designed mechanically circumference-expandable against the back-spring force.

2. Endoscope optics according to claim 1, **characterized in that** the tube (30, 40, 50, 60, 80) is designed extending over the secured lenses (4, 85, 86).

3. Endoscope optics according to claim 1, **characterized in that** the tube is designed in form of a hose (30) of an elastically circumference-expandable material.

4. Endoscope optics according to claim 1, **characterized in that** the tube is designed in form of a cable grip (40) consisting of fibres (41) being crosswise diagonally running interwoven or interlaced.

5. Endoscope optics according to claim 1, **characterized in that** the tube (26, 50, 60, 80) is made from an elastically bendable material, having a slot (27, 51, 61) connecting continuously the ends of the tube.

6. Endoscope optics according to claim 5, **characterized in that** the tube consists of a flat wire helix (60).

7. Endoscope optics according to claim 5, **characterized in that** the tube (26, 50, 60, 80) consist of metal.

8. Endoscope optics according to claim 5, **characterized in that** the slot (51) of the tube (26, 50, 80) is running parallel to the axis.

9. Endoscope optics according to claim 8, **characterized in that** the width of the slot (51) of tube (80) is smaller than 180° angle at circumference.

10. Endoscope optics according to claim 1, **characterized in that** the tube (30, 40, 50, 60, 80) accommodates all the lenses (4, 85, 86) of the endoscope optics.

11. Endoscope optics according to claim 1, having a distally provided objective (85, 86), **characterized in that** the tube (80) accommodates at least the objective (85, 86).

12. Endoscope optics according to claim 1, comprising a system tube (2) accommodating the lenses (4, 85, 86), **characterized in that** the circumferentially expendable tube (6, 60, 80) is arranged with radial clearance within the system tube (2).

## Revendications

1. Optique endoscopique (1) dotée de lentilles (4, 85, 86) et d'un dispositif d'arrêt (6, 26, 30, 40, 50, 60, 80) assurant le positionnement des lentilles (4, 85, 86) contre un désajustement en cas de contraintes par chocs, lequel dispositif est réalisé sous forme d'un tube (6, 26, 30, 40, 50, 60, 80) qui enveloppe conjointement les extrémités contiguës de deux lentilles (4, 85, 86) à au moins un point de transition et peut être évasé de sorte à exercer une force permanente de retour élastique, **caractérisée en ce que** la circonférence du tube (6, 26, 30, 40, 50, 60, 80) peut être mécaniquement évasée contre la force de retour élastique.

2. Optique endoscopique selon la revendication 1, **caractérisée en ce que** le tube (30, 40, 50, 60, 80) est réalisé de façon à s'étendre sur toute la longueur des lentilles (4, 85, 86) stabilisées.

3. Optique endoscopique selon la revendication 1, **caractérisée en ce que** le tube est réalisé sous forme de tuyau (30) en un matériau dont la circonférence est élastiquement extensible.

4. Optique endoscopique selon la revendication 1, **caractérisée en ce que** le tube est réalisé sous forme de manchon rétractable (40) constitué d'un tissu ou réseau de fibres (41) entrecroisées obliquement.

5. Optique endoscopique selon la revendication 1, **caractérisée en ce que** le tube (26, 50, 60, 80) est réalisé en un matériau élastique flexible et doté, de bout en bout, d'une fente (27, 51, 61) joignant une extrémité à l'autre.

6. Optique endoscopique selon la revendication 5, **caractérisée en ce que** le tube est réalisé sous forme de spirale de fil plat (60).

7. Optique endoscopique selon la revendication 5, **caractérisée en ce que** le tube (26, 50, 60, 80) est réalisé en métal.

8. Optique endoscopique selon la revendication 5, **caractérisée en ce que** la fente (51) du tube (26, 50, 80) est orientée parallèlement à l'axe du tube.

9. Optique endoscopique selon la revendication 8, **caractérisée en ce que** la largeur de la fente (51) du tube (80) est inférieure à un angle inscrit de 180°.

10. Optique endoscopique selon la revendication 1, **caractérisée en ce que** le tube (30, 40, 50, 60, 80) sert de logement à toutes les lentilles (4, 85, 86) de l'optique endoscopique.

11. Optique endoscopique selon la revendication 1 dotée d'un objectif (85, 86) distal, **caractérisée en ce que** le tube (80) sert au moins de logement à l'objectif (85, 86).

12. Optique endoscopique selon la revendication 1 comportant un tube (2) enveloppant les lentilles (4, 85, 86) du système optique, **caractérisée en ce que** le tube (6, 60, 80) pouvant être évasé est logé dans le tube (2) du système optique avec un certain jeu radial.
